# EUROPÄISCHE PATENTSCHRIFT

(11) **EP 1 936 362 B1**
(45) Veröffentlichungstag und Bekanntmachung des Hinweises auf die Patenterteilung: **18.03.2020**
(21) Anmeldenummer: 06026395.1
(22) Anmeldetag: 20.12.2006
(51) Int. Cl.: G01N 21/86, G01N 21/84, A61B 5/145, A61B 5/1455

(54) **Testelement mit Referenzierung**
Test element with referencing
Elément de test avec référencement

(43) Veröffentlichungstag der Anmeldung: 25.06.2008
(73) Patentinhaber: Roche Diabetes Care GmbH, 68305 Mannheim (DE); F. Hoffmann-La Roche AG, 4070 Basel (CH)
(72) Erfinder: Petrich, Wolfgang, 76669 Bad Schönborn (DE); Hoenes, Joachim, 64673 Zwingenberg (DE); Krämer, Uwe, 68549 Ilvesheim (DE)
(74) Vertreter: Altmann Stößel Dick Patentanwälte PartG mbB

(56) Entgegenhaltungen:
- EP-A- 1 359 409
- EP-A1- 1 710 565
- EP-A2- 0 469 377
- WO-A-2006/096210
- US-A- 4 125 372
- US-A- 5 132 097
- US-A- 5 485 527
- US-A1- 2005 157 304
- US-B1- 6 249 593

## Beschreibung

### Technisches Gebiet der Erfindung:

Die Erfindung betrifft die Konzentrationsbestimmung eines Analyten in Flüssigkeit.

### Stand der Technik

Die Konzentrationsbestimmung verschiedener Analyten in physiologischen Proben ist von wachsender Bedeutung in unserer Gesellschaft. Die Untersuchung solcher Proben findet in verschiedenen Anwendungsbereichen statt, z. B. in klinischen Labors oder im "Home-Monitoring". Hierbei sind die Ergebnisse von großer Bedeutung, bei der Handhabung verschiedener Krankheiten. Hierzu zählt vor allem auch die Glukosemessung beim Diabetes Management sowie die Cholesterinmessung bei Herz- und Gefäßkrankheiten. Die medizinische Blutdiagnostik setzt stets die Gewinnung einer Blutprobe des zu untersuchenden Individuums voraus.

Die nach dem Stechvorgang durchgeführte Analytik wird häufig in einem kleinen, tragbaren Messgerät, einem so genannten "Handheld device" durchgeführt, in dem mit Blut benetzte Testelemente analysiert werden. Eine große Bedeutung haben diese Handheld devices vor allem in der Diagnostik von Diabetes Erkrankungen. Die Messung in diesen Geräten wird vor allem elektrochemisch oder optisch durchgeführt. Bei den optisch basierten Messungen, wird die Probe mit Licht beleuchtet und das reflektierte Licht detektiert, um die Analytkonzentration zu bestimmen. Hierzu werden vor allem Testelemente wie Teststreifen verwendet, die auf einem Nachweisbereich, der Teil des Testelementes ist, mit der Probe, wie Blut oder interstitielle Flüssigkeit benetzt werden. Die Probe reagiert anschließend mit den Reagenzien, die in oder auf dem Nachweisbereich aufgebracht sind. Dies kann zu einer Farbveränderung führen oder aber auch bei einer elektrochemischen Reaktion zu Ladungsänderungen, die dann detektiert werden können.

Bei der optischen Auswertung von Nachweisbereichen auf die nur kleine Probemengen aufgebracht werden, ist es von großer Bedeutung, dass die benetzte Fläche des Nachweisbereiches optimal ausgewertet wird. Dies ist neben der ausreichenden Beleuchtung und Detektion durch die Verwendung von Referenzbereichen möglich. Die Referenzierung kann auf unterschiedliche Weise durchgeführt werden. So kann ein vom Nachweisbereich separierter Referenzbereich herangezogen werden wodurch technische Effekte wie Eigenschaften der Lichtquelle oder des Detektors sowie bestimmte Eigenschaften des Testelementes ausgeglichen werden können. Um eine Referenzierung auf die Probeneigenschaften vornehmen zu können, sollte der Referenzbereich ähnlich zum Nachweisbereich aufgebaut sein.

Im Dokument US2004/071331-A1 wird ein reflektometrisches System zur Bestimmung von Lichtintensitäten eines gefärbten Fleckes benutzt, wobei der den Fleck umgebenden Umgebungsbereich zur Referenzierung herangezogen wird. Der Nachteil dieser Methode ist, dass das Hintergrundsignal zur Referenzierung nicht von der Probe benetzt ist und deswegen andere optische Eigenschaften aufweist als der mit Probe benetzte Bereich.

In der europäischen Patentanmeldung EP 0 469 377 wird ein Verfahren beschrieben, eine Analytkonzentration in einer Probe zu bestimmen, wobei zwei Teilbereiche mit unterschiedlicher Bindungsaktivität zu dem Analyten in der Probe unterschieden werden. In dem Teilbereich mit höherer Bindungsaktivität ist ein Bindungspartnerträger fixiert, der den Analyten bei Aufgabe der Probe bindet. In dem Teilbereich mit weniger Bindungsaktivität gegenüber dem Analyten ist kein Bindungspartner auf dem Träger fixiert. Der Bereich mit weniger Bindungsaktivität wird zur Referenzierung herangezogen. Nachteil dieses Analysesystems ist, dass mit dieser Analysemethode nur Reaktionen durchgeführt und referenziert werden können, die eine Bindung des Analyten an einen Träger fixierten Bindungspartner voraussetzen, sodass keine Diffusion des Analyten mehr möglich ist. Die Bestimmung des Analyten ist auf gebundene Moleküle beschränkt.

Das US Patent mit der Nummer US 6,249,593 B1 beschreibt ein System zur Detektion eines Analyten auf einem Testträger. Der Analyt wird an einen auf den Testträger immobilisierten Rezeptor gebunden. Ein Teil des Testträgers, das keinen immobilisierten Rezeptor enthält wird als Referenz herangezogen. Auch in diesem Dokument setzt die Bestimmung des Analyten eine Bindung an eine immobilisierte Komponente voraus.

In EP 1710565-A1 wird ein System zur Auswertung eines Testelements, insbesondere eines Testelements zur medizinischen Diagnose, vorgeschlagen sowie eine Haltevorrichtung zur Auswertung eines Testelements. Weiterhin wird ein Kalibrierelement zum Kalibrieren des Systems vorgeschlagen sowie ein Verfahren zum Durchführen einer Diagnose,

Das Patent EP 1 359 409 B1 beschreibt ein analytisches Gerät zur Konzentrationsbestimmung eines Analyten in einer physiologischen Probe, welches unter Verwendung eines Teststreifens mit einem Testfeld arbeitet. Das Gerät umfasst wenigstens eine Lichtquelle, einen Detektor zur Detektion reflektierten Lichts, das von einer Vielzahl verschiedener Bereiche des Teststreifens ausgeht, Mittel zur Bestimmung einer ausreichenden Benetzung der vorgenannten Bereiche des Teststreifens auf Basis des reflektierten Lichts, sowie Mittel zur Konzentrationsbestimmung auf Basis des reflektierten Lichts, wobei Bereiche mit ungenügender Benetzung nicht für die Konzentrationsbestimmung herangezogen werden.

Aus den Nachteilen des Standes der Technik ergibt sich die Aufgabe, ein Analysesystem zur Bestimmung der Konzentration eines Analyten zu entwickeln, das keine irreversible Bindung des Analyten an den Testträger voraussetzt.

Weiterhin ist eine Aufgabe, ein Analysesystem vorzuschlagen, das eine genauere Bestimmung von Analyten mit sehr kleinen Probevolumina ermöglicht.

Diese Aufgaben werden gelöst durch ein System zur Bestimmung der Konzentration eines Analyten in einer Flüssigkeit durch Absorptionsmessung, beinhaltend ein Testelement mit einem Nachweisbereich, der mindestens einen Reaktionsbereich mit Reagenzien zum Nachweis des Analyten enthält, die bei Reaktion mit dem Analyten eine Veränderung des Absorptionsverhaltens verursachen und der Nachweisbereich mindestens einen Referenzbereich enthält. Weiterhin enthält das System eine Detektionseinheit zur räumlich aufgelösten Detektion von Lichtintensitäten, die vom Nachweisbereich empfangen werden und einer Auswerteeinheit zur Auswertung von Signalen der Detektionseinheit. Das System ist dadurch charakterisiert, dass der mindestens eine Referenzbereich so beschaffen ist, dass sich das Absorptionsverhalten bei Benetzung im Wesentlichen nicht verändert.

Der Nachweisbereich kann dabei verschiedene Formen annehmen. Er kann beispielsweise eckig oder rund sein. Eine bevorzugte Form des Nachweisbereiches ist quadratisch oder rechteckig. Dabei weist der Nachweisbereich eine Länge und eine Breite auf. Die von der Länge und Breite aufgespannte Fläche weist sowohl mindestens einen Reaktionsbereich als auch mindestens einen Referenzbereich auf.

Der mindestens eine Reaktionsbereich unterscheidet sich von dem mindestens einen Referenzbereich dadurch, dass in dem Referenzbereich im Wesentlichen keine Veränderung des optischen Absorptionsverhaltens durch den Analyten erfolgt. Dies wird bevorzugter weise dadurch ermöglicht, dass der Referenzbereich keine Reagenzien aufweist, die mit dem Analyten in Reaktion treten können. Eine weitere Möglichkeit die Änderung des Absorptionsverhaltens in dem mindestens einen Referenzbereich möglichst gering zu halten, ist die Vermeidung der Benetzung des mindestens einen Referenzbereiches mit der Probe.

Bevorzugter weise wird das nicht in Kontakt treten der Probe mit dem Referenzbereich durch die geometrische Anordnung des Referenzbereiches in Relation zum Reaktionsbereich erreicht. So kann beispielsweise eine Barriere zwischen den beiden Bereichen vorhanden sein. Neben der Abtrennung des Referenzbereiches durch eine Barriere kann der Referenzbereich auch in der Höhe versetzt zu dem Reaktionsbereich angeordnet sein, sodass bei Aufgabe auf den Nachweisbereich der Referenzbereich nicht mit Probe in Kontakt gebracht wird. Bei einer automatisierten Applikation der Probe auf den Nachweisbereich, wie dies beispielsweise durch Verwendung einer Kapillare oder saugfähiger Materialien möglich ist, kann der Referenzbereich soweit von der Aufgabeposition entfernt liegen, dass durch den gewählten Abstand zwischen Referenzbereich und Aufgabeort die Probenflüssigkeit nicht mit dem Referenzbereich in Kontakt treten kann. In einer bevorzugten Ausführungsform wird flüssige Probe, die zum Beispiel von einer Nadelstruktur aufgenommen wurde durch gezielte Kontaktierung des Nachweisbereiches mit der Nadelstruktur appliziert. Durch die gezielte (z.B. automatisierte) Kontaktierung des Nachweisbereiches mit der Nadelstruktur, kann erreicht werden, dass nur ausgewählte Bereiche des Nachweisbereiches mit Flüssigkeit in Kontakt treten.

In einer weiteren bevorzugten Ausführungsform tritt die Probe sowohl mit dem Reaktionsbereich als auch mit dem Referenzbereich in Kontakt. Der Reaktionsbereich ist so beschaffen, dass sich das Absorptionsverhalten bei Benetzung im Wesentlichen nicht verändert. Die minimale Änderung des Absorptionsverhaltens in den Referenzbereichen ist auf die Eigenschaften der Probe zurückzuführen, die als Flüssigkeit (wie beispielsweise Serum, Blut oder Urin) auf den Nachweisbereich gegeben wird. Zu diesen Eigenschaften zählt z.B. der Brechungsindex, der sich auf dem Nachweisbereich bei Aufgabe einer Flüssigkeit auf den Nachweisbereich ändert, da die Luft, die durch die Flüssigkeit verdrängt wird, einen anderen Brechungsindex besitzt als die aufgegebene Flüssigkeit. Aber auch feste Bestandteile oder Farbstoffe sowie andere Bestandteile der Flüssigkeitsprobe gehören unter anderem zu den Eigenschaften, die das Absorptionsverhalten beeinflussen, ohne dass hieraus auf die Anwesenheit eines Analyten zu schließen wäre. Da die Änderung des Absorptionsverhaltens in dem Referenzbereich dabei nur einen Bruchteil der Absorptionsänderung durch die Änderung des Analyten im Reagenzbereich aufweist, kann von einem im Wesentlichen unveränderten Adsorptionsverhalten gesprochen werden. Dabei beträgt die Änderung des Absorptionsverhaltens in den Referenzbereichen nicht mehr als 30 %, bevorzugter weise nicht mehr als 5 %, besonders bevorzugt nicht mehr als 1 % der ursprünglichen Absorption.

In einer bevorzugten Ausführungsform sind mehrere Reaktionsbereiche und mehrere Referenzbereiche in zwei Dimensionen alternierend angeordnet. In zwei Dimensionen alternierend bedeutet, dass der Nachweisbereich auf der Fläche, auf der die Reaktionsbereiche und die Referenzbereiche angeordnet sind, abwechselnd mindestens ein Reaktionsbereich und mindestens ein Referenzbereich zu finden ist. Dies ist unabhängig von der Ebene von welcher auf die Fläche geschaut wird. Die zwei Dimensionen spannen dabei die Grundfläche des Nachweisbereiches auf und die Achsen der beiden Dimensionen sind rechtwinklig zueinander angeordnet. Dies hat zur Folge, dass sowohl in die eine Dimension mindestens ein Reaktionsbereich und mindestens ein Referenzbereich angeordnet ist als auch in die zweite Dimension. Die einzelnen Bereiche können dabei unterschiedliche Formen und Größen aufweisen.

Bei Aufgabe der Probenflüssigkeit auf den Nachweisbereich findet mindestens in dem mindestens einen Reaktionsbereich eine Reaktion des Analyten mit Reagenzien zum Nachweis des Analyten statt. Bei dieser Reaktion des Analyten mit den Reagenzien ändert sich das optische Absorptionsverhalten des mit Probeflüssigkeit benetzten mindestens einen Reaktionsbereiches. In den wichtigsten praktischen Anwendungen enthalten die Reagenzien mindestens ein Enzym, das bevorzugter weise auf dem Nachweisbereich immobilisiert ist. Ein Teil der Reaktionsprodukte dieser enzymatischen Reaktion kann als Zwischenprodukt für eine weitere enzymatische Reaktion dienen oder ein Endprodukt der enzymatischen Reaktion kann selbst das Absorptionsverhalten in dem Reaktionsbereich verändern. In beiden Fällen (Endprodukt oder Zwischenprodukt) bildet sich im Verlauf der Reaktion ein Farbstoff, der das Absorptionsverhalten des Reaktionsbereiches verändert. Die Änderung des optischen Absorptionsverhaltens findet aufgrund der Reaktion des Analyten mit mindestens einem Reagenz in dem Reaktionsbereich statt. Die Absorption bei der eingestrahlten Wellenlänge kann sich dabei erhöhen oder erniedrigen. Bevorzugter Weise erhöht sich die Absorption, da in einer bevorzugten Reaktion ein Farbstoff gebildet wird, der Licht bei der eingestrahlten Wellenlänge absorbiert. Das Licht, das von dem Nachweisbereich reflektiert oder transmittiert wird, kann mit Hilfe eines Detektors detektiert werden. Im Folgenden wird ein System beschrieben, bei dem die Veränderung des Absorptionsverhaltens aufgrund Bildung eines Farbstoffes stattfindet.

In dem bzw. den Referenzbereichen findet mindestens die, das Absorptionsverhalten ändernde Reaktion aus dem Reaktionsbereich nicht statt. Dies kann verschiedene Ursachen haben, die beispielhaft im Folgenden beschrieben werden:
1. Es befindet sich keinerlei Enzym in den Referenzbereichen.
2. Das mindestens eine Enzym wurde in den Referenzbereichen deaktiviert.
3. Die Farbbildende Reaktion läuft im Referenzbereich nicht ab.
4. Eine zusätzliche Beschichtung des Referenzbereiches verhindert mindestens teilweise die Benetzung des Referenzbereiches mit Probe.
5. Der Referenzbereich wird nicht mit Probe benetzt.

Auf diese Weise kann der Referenzbereich im Aufbau und Verhalten gegenüber dem Analyten sehr ähnlich zum Reaktionsbereich gestaltet werden. Jedoch findet im Referenzbereich keine optische Absorptionsveränderung durch den Analyten statt. Durch die ersten drei Varianten kann gewährleistet werden, dass die Eigenschaften der Probe bei der Referenzierung mit berücksichtigt werden, was die Genauigkeit der Messergebnisse erhöht. Diese sind besonders bevorzugt in Systemen einsetzbar die möglichst kleine Testelemente bzw. Nachweisbereiche aufweisen, da die Messsignale bei miniaturisierten Systemen sehr klein und damit fehleranfällig sind. Durch die Referenzierung auf möglichst identische Bereiche des Nachweisbereiches, bei der sowohl Unregelmäßigkeiten des Testelementes als auch der Probe berücksichtigt werden, kann die Probemenge sehr klein sein, um noch ausreichend genau ausgewertet zu werden.

Um eine Diffusion des gebildeten Farbstoffes von den Reagenzbereichen in Referenzbereiche zu verhindern, kann ein Farbstoff gewählt werden, der nach seiner Entstehung durch die Reaktion des Analyten mit den Reagenzien wasserunlöslich ist. Das hat zur Folge, dass der Farbstoff in wässriger Lösung (wie beispielsweise Blut) ausfällt und keine Diffusion des Farbstoffes in benachbarte Bereiche, wie beispielsweise die Referenzbereiche, stattfinden kann.

Die Anordnung und/oder Form der Reagenz- und der Referenzbereiche kann zufällig gewählt werden oder gleichmäßig erfolgen. In einer bevorzugten Ausführungsform besitzen die Reaktions- und Referenzbereiche unterschiedliche Größen, besonders bevorzugt ist der mindestens eine Referenzbereich kleiner als der mindestens eine Reaktionsbereich. In einer weiteren bevorzugten Ausführungsform sind mehrere Reagenz- und Referenzbereiche regelmäßig angeordnet. Dabei können Reaktions- und Referenzbereiche die gleiche Größe und / oder das gleiche Volumen aufweisen. Um möglichst kleine Probevolumina auswerten zu können, sollte die Fläche der Reaktions- und Referenzbereiche möglichst klein gewählt werden. Da der Nachweisbereich in seinen Dimensionen nur wenige Quadratmillimeter groß ist, legt auch die bevorzugte Fläche von Reaktions- bzw. Referenzbereichen bei wenigen Quadratmillimetern. Die kleinste mögliche Größe eines Nachweisbereiches hängt einerseits von der Wahl des Herstellverfahrens für Reaktions- und Referenzbereich und andererseits von der Genauigkeit der Detektion ab. Wird ein hoch auflösendes Detektionssystem benutzt, können sehr kleine Bereiche optisch unterschieden werden. In einer bevorzugten Ausführungsform ist die Fläche des Reaktions- bzw. des Referenzbereiches < 1 mm². Um eine ausreichend hohe Genauigkeit bei der Analyse des Analyten zu erreichen, sollte mindestens ein Teil eines Reaktionsbereiches und ein Teil eines Referenzbereiches mit Probe benetzt werden. Je kleiner die Bereiche gewählt werden, desto weniger Probenflüssigkeit wird zum Nachweis des Analyten benötigt, um sowohl genügend Signal eines ausreichend benetzten Reaktionsbereiches als auch eines ausreichend benetzten Referenzbereiches zu erhalten. Wenn die Reaktionsbereiche und Referenzbereiche alternierend in 2 Dimensionen angeordnet sind, wie dies für eine bevorzugte Ausführungsform beschrieben wurde, reicht die Benetzung eines einzigen Reaktions- und Referenzbereiches aus, um eine Konzentrationsbestimmung des Analyten vorzunehmen. Um hierfür eine möglichst kleine Probe vermessen zu können, können die Reaktions- bzw. Referenzbereiche möglichst klein gewählt werden, sodass bei Aufgabe der Probe mindestens ein Reaktionsbereich und ein Referenzbereich ausreichend benetzt werden.

Zur Herstellung eines Testelementes mit verschiedenen Reaktions- und Referenzbereichen, die in zwei Dimensionen alternierend angeordnet sind, sind vor allem Druckverfahren und/oder Rakelverfahren bevorzugt. Dabei kann der Nachweisbereich durchgehend mit Reagenzien beschichtet werden, die anschließend durch Laserbestrahlung und/oder Veränderung ihrer chemischen Zusammensetzung zum Teil in Referenzbereiche umgewandelt werden können.

### Aufbau Referenzbereich

Um eine möglichst gute Referenzierung zu erreichen, sollte der Referenzbereich so ähnlich wie möglich zum Reaktionsbereich ausgestaltet werden. Bei einer enzymatischen Reaktion des Analyten mit dem Reagenz können die Reaktionsprodukte in der Regel vom Reaktionsort weg diffundieren, da der Analyt nicht an den Träger gebunden wird. Aus diesem Grund werden Reaktionsbereiche und Referenzbereiche meistens nicht in unmittelbarer Nähe zueinander auf das Testelement aufgebracht. Die unmittelbare Nähe von Reaktions- und Referenzbereich ist nur möglich, wenn der Analyt an einen Träger fixiert wird, wie dies bei immunologischen Reaktionen stattfindet, wie in US 6,249,593 B1 beschrieben wird, wobei der Analyt zusammen mit dem Nachweisreagenz an den immobilisierten Reaktionspartner gebunden wird. Die Diffusionsprozesse, die bei Analysesystemen auftreten, bei denen das Reaktionsprodukt nicht an einen immobilisierten Reaktionspartner gebunden wird, können dazu führen, dass das Reaktionsprodukt (z.B. Farbstoff) aus dem Reaktionsbereich in den Referenzbereich diffundiert. Hierdurch wird eine Referenzierung erschwert oder unmöglich gemacht. Um eine räumlich Trennung von Reaktionsbereich und Referenzbereich zu vermeiden und damit eine erhöhte Aufgabe von Probe zu vermeiden, können verschiedene Maßnahmen getroffen werden. Zum einen kann das Enzym im Reaktionsbereich immobilisiert werden, was eine räumliche Begrenzung der Reaktionsprodukte zur Folge hat. Außerdem kann der sich bildende Farbstoff wasserunlöslich ausgestaltet werden. Hierdurch wird die Veränderung des Absorptionsverhaltens auf die Reaktionsbereiche beschränkt. Auf diese Weise können die Reaktionsbereiche und Referenzbereiche in unmittelbarer Nähe angeordnet werden und sehr klein ausgestaltet werden, da eine Diffusion von zu detektierenden Reagenzien verhindert wird.

Da im Referenzbereich keine das Absorptionsverhalten ändernde Reaktion stattfindet, kann bei Benetzung der Referenzbereiche eine Diffusion des Analyten bzw. von Zwischenprodukten in die Reaktionsbereiche stattfinden, da mindestens der Farbstoff bildende Reaktionsschritt in den Referenzbereichen nicht mehr stattfindet. Um diese Diffusion über den ganzen Nachweisbereich vergleichbar zu gestalten, kann eine in 2 Dimensionen alternierende Anordnung der Referenz- und Reaktionsbereiche gewählt werden. Hierbei ist die Größe und Form der Bereiche nicht limitiert. Eine gleichmäßige Verteilung von Referenz- und Reaktionsbereichen wird jedoch bevorzugt, um eine vergleichbare Diffusion in allen Bereichen und damit eine Vergleichbarkeit der Bereiche zu gewährleisten. Dies ist beispielsweise in einer schachbrettartigen Anordnung von Referenz- und Reaktionsbereichen möglich.

Alternativ zu der Inaktivierung von Enzymen durch Laser oder dem Nichtaufbringen von Enzym in die Referenzbereiche kann die Reaktion des Analyten mit Reagenz in den Referenzbereichen dadurch verhindert werden, dass in einen Teil des Referenzbereiches eine Substanz eingebracht wird, die das Eindringen des Analyten in diesen Bereich verhindert. Dies kann eine Membran sein, die zwar durchlässig für Wassermoleküle ist, aber das Eindringen größerer Moleküle, wie dem Analyten, verhindert. Alternativ kann auch das Eindringen von Flüssigkeit generell verhindert werden. Dies kann beispielsweise durch eine hydrophobe Substanz geschehen, wie beispielsweise ein hydrophober Kunststoff oder ein Kunstharz, der das Eindringen von Flüssigkeit und damit auch von Analyt verhindert. Solche Kunststoffe und Kunstharze sind im Stand der Technik hinlänglich bekannt.

### Enzym/Coenzym Zusammensetzung

Die Wahl der Reagenzien hängt von der Gestalt des Analyten ab, dessen Konzentration bestimmt werden soll. Als Analyten können alle in einer Flüssigkeit löslichen Substanzen dienen. Bevorzugter weise ist dieser Analyt in einer Körperflüssigkeit gelöst, wie z. B. Zielmoleküle wie Cholesterin, Glucose, Triglyceride, Harnstoff, Harnsäure oder HbA_{1c}. Hierzu können verschiedene Enzyme, wie beispielsweise Dehydrogenasen (Gluc - DH), Oxidoreduktasen (z.B. GlucDOR) zusammen mit Coenzymen, wie Flavin-(z.B. FAD/FADH), Nicotin-(z.B. NAD/NADH) oder Chinonderivate (z.B. Q, PQQ) verwendet werden. Diese Enzym-Coenzym Systeme sind hinlänglich aus dem Stand der Technik bekannt, wie beispielsweise in der Patentanmeldung US 2005 0214891 beschrieben. In einer bevorzugten Ausführungsform ist Glucose der Analyt und kann beispielsweise durch folgende enzymatische Reaktionen nachgewiesen werden:
1. mittels GOD-FAD, POD-Häm und Ausfällen von Leucofarbstoff am Ort der immobilisierten POD.
2. mittels Glucose-Dye Oxidoreductase (GlucDOR-PQQ), Mediator und immobilisierter Phosphormolybdänsäure. pyrroloquinoline quinone (PQQ)
3. mittels Gluc-DH- unterstützter Umsetzung von NAD in NADH mit anschließender Umsetzung von Tetrazoliumsalz mittel immobilisierter Diaphorase, wobei in letzterem Schritt Formazan ausfällt.

Eine Komponente in der Reaktionskette ist bevorzugter weise immobilisiert. Besonders bevorzugt ist das die Komponente, die am Ende der Reaktionskette steht, aus der sich die das Absorptionsverhalten ändernde Substanz bildet. So ist beispielsweise das glucosespezifische Enzym, wie GOD, GlucDOR oder GlucDH in den Reaktionsbereichen alternierend zu den Referenzbereichen in einem schachbrettartigen Muster auf einer Folie des Nachweisbereiches immobilisiert.

Um verschiedene Analyten gleichzeitig in einer Probe auf einem Testelement zu vermessen, können in verschiedene Reaktionsbereiche unterschiedliche Enzyme eingebracht werden.

### Herstellverfahren

Die Strukturierung des Nachweisbereiches in Referenz- und Reaktionsbereiche kann neben anderen herkömmlichen Herstellungsverfahren, wie in US 6,592,815 oder US 7,008,799 beschrieben, beispielsweise mit Hilfe von verschiedenen Druckverfahren hergestellt werden:
- Siebdruck
- Offsetdruck
- Tintenstrahldruck
- Rakel

Mit Hilfe dieser Verfahren ist es möglich in einem Schritt die Oberfläche des Nachweisbereiches, der sich auf einem Trägermaterial befindet, wie es üblich für den Aufbau von Testelementen ist, so zu strukturieren, dass sie verschiedene Bereiche mit unterschiedlichen Funktionalitäten aufweist. So können die Reaktionsbereiche mit allen nötigen Reagenzien bedruckt werden, während in den Referenzbereichen ein Teil der Reagenzien nicht aufgebracht wird. Alternativ kann der Nachweisbereich analog zu den Reaktionsbereichen bedruckt werden, wobei anschließend eine Deaktivierung der Referenzbereiche erfolgt, sodass die Enzyme, in den Referenzbereichen mit dem Analyten nicht mehr reagieren können. Diese Deaktivierung kann beispielsweise durch Bestrahlung mit Laserlicht stattfinden oder durch gezielte Aufgabe von inaktivierenden Substanzen wie beispielsweise Säuren oder Basen. Alternativ kann den Referenzbereichen auch eine Substanz zugesetzt werden, die irreversibel an das Enzym bindet und dieses für eine Reaktion mit dem Analyten blockiert.

### Detektion

Die Beleuchtung des Nachweisbereiches kann durch eine oder mehrere Lichtquellen geschehen. Dabei kann der Nachweisbereich homogen ausgeleuchtet sein oder nur in Teilbereichen. Bei der Verwendung von nur einer Lichtquelle kann eine homogene Ausleuchtung des Nachweisbereiches durch die Verwendung eines Milchglases oder anderer streuender Einheiten verbessert werden.

Eine Alternative zur Beleuchtung des Nachweisbereiches mit mindestens einer Lichtquelle, ist die Nutzung des Umgebungslichtes (Sonnenlicht oder künstliche Beleuchtung) zur Ausleuchtung des Nachweisbereiches. Da das Umgebungslicht multispektral ist, kann zur Detektion nur eines bestimmten Wellenlängenbereiches ein Filter zwischen Testelement und Detektor eingesetzt werden.

Alternativ kann das System zur sequentiellen Beleuchtung des Testelementes mit verschiedenen Beleuchtungseinheiten versehen sein. Dies ist jedoch nicht zwingend nötig. Als Lichtquelle kann beispielsweise eine einfache Laserdiode kombiniert mit einem Reflektor der durch Mikromechanik verstellbar ist, dienen. Mit Hilfe des Reflektors kann der Lichtstrahl das Testelement lückenlos abrastern. Alternativ kann ein Laser-Array eingesetzt werden, bevorzugt ein VCSEL-Array (Vertical Cavity Surface Emitting Laser). Jeder Laser im Array ist hierbei einzeln adressierbar. Die VCSEL bieten den Vorteil, dass das Licht mit geringer Strahldivergenz abgestrahlt wird. Diese Laserstrukturen weisen eine Strahldivergenz von etwa 5-8° auf. Auf diese Weise ist nicht nur eine kleine Fläche zu bestrahlen, zusätzlich ist die Lichtmenge auf dieser Fläche sehr hoch.

Die Beleuchtungseinheit kann aus einer monochromen oder multispektralen, kohärenten oder inkohärenten Strahlungsquelle bestehen. Die Strahlung aus der Beleuchtungseinheit dient dazu in den Nachweisbereich einzudringen, um die Farbreaktion eines Reagenz mit dem Analyt zu messen. Vorzugsweise besteht die Beleuchtungseinheit aus einer oder mehreren LED's deren Licht am Probenort entweder für eine speziell gewählte räumliche Intensitätsverteilung oder für eine homogene Ausleuchtung sorgt. In einer bevorzugten Ausführungsform wird Licht mit einer Wellenlänge von ca. 660 nm benutzt. Dies kann durch die Wahl der Lichtquelle umgesetzt werden oder durch den Einbau von abbildenden Einheiten wie Filter, die nur für einen definierten Wellenlängenbereich lichtdurchlässig sind.

Zwischen der Beleuchtungseinheit und dem Nachweisbereich kann eine Abbildungseinheit eingebaut sein. Diese Abbildungseinheit besteht aus optischen Elementen wie Linsen, Filtern, Spiegeln, Blenden, Prismen, Lichtleitenden oder holographischen Elementen. Dadurch wird eine Beleuchtung des Nachweisbereichs gewährleistet. Eine weitere Abbildungseinheit dient zur Projektion des bestrahlten Probenkörpers auf die Detektionseinheit. Diese Abbildungseinheit besteht ebenfalls aus abbildenden optischen Elementen wie Linsen, Filtern, Spiegeln, Prismen, Blenden, Lichtleitenden oder holographischen Elementen. Wahlweise kann ein mikrooptisches Linsenarray verwendet werden bei dem jedes Einzelelement abgegrenzte räumliche Bereiche des Testelements auf Einzelelemente der Detektionseinheit abbildet. Bei Verwendung einer multispektralen Lichtquelle ist es sinnvoll, einen Filter vor dem Detektor bzw. vor das Testelement anzubringen.

Detektionseinheiten, zur Verwendung in der vorliegenden Erfindung, können aus einem flächigen oder zeilenförmigen Element bestehen, das die orts- wie auch die zeitaufgelöste Messung der gestreuten Strahlung, die vom Nachweisbereich abgebildet wird, ermöglicht. Vorzugsweise handelt es sich bei diesem Element um ein zweidimensionales CMOS-Array, ein CCD Array oder ein zeilenförmiges Diodenarray, bei dem die ortsaufgelöste Abbildung des Nachweisbereiches mittels eines Scanvorganges durchgeführt wird. Häufig kann auch eine einfache Photodiode ohne Ortsauflösung hinreichend sein. Diese kann beispielsweise in Kombination mit einer ortsaufgelösten Bestrahlung des Nachweisbereiches benutzt werden.

### Messschritte

Bei der Durchführung einer Messung werden vom Patienten und vom System folgende Schritte ausgeführt:
- Aufbringen der Probe auf den Nachweisbereich eines Testelementes,
- ortsaufgelöstes Vermessen der reflektierten Lichtintensitäten vom Nachweisbereich
- Bestimmung der Analytkonzentration mit Hilfe der von mindestens einem Reaktionsbereich und von mindestens einem Referenzbereich vermessenen Lichtintensitäten.

Dabei wird ein Testelement verwendet, das mindestens einen Reaktionsbereich und mindestens einen Referenzbereich beinhaltet, wobei bei Benetzung des Referenzbereiches im Wesentlichen keine Veränderung des Absorptionsverhaltens durch den Analyten und/oder die Probe erfolgt. Zur Detektion können Detektionssysteme verwendet werden wie sie bereits beschrieben wurden.

Das in dieser Anmeldung beschriebene System bzw. Verfahren zur Bestimmung der Konzentration eines Analyten in einer Flüssigkeit bietet den Vorteil, dass keine weitere Referenzierung im Gerät nötig ist. Es braucht also kein zusätzliches Referenzfeld im System oder auf dem verwendeten Testelement angebracht werden, um eine ausreichende Genauigkeit der Messung zu erreichen.

Durch die Anordnung von Referenzbereichen und Reaktionsbereichen ist es möglich, diese Bereiche simultan auszuleuchten und/oder zu detektieren und/oder auszuwerten. Es ist keine Separate Ausleuchtung oder Detektion der verschiedenen Bereiche nötig.

Darüber hinaus gibt es für den Patient keine Notwendigkeit darauf zu achten wie er die Probe auf dem Testelement appliziert, da durch die Größe und Form des mindestens einen Referenz- bzw. mindestens einen Reaktionsbereiches gewährleistet ist, dass mindestens ein Teil des Referenz- und ein Teil des Reaktionsbereiches zur Analyse herangezogen werden kann.

### Figurenbeschreibung

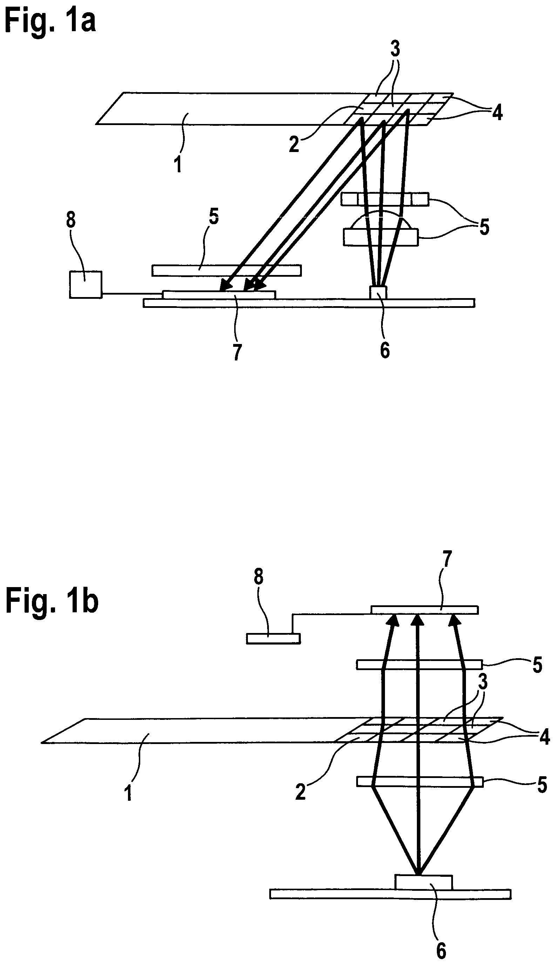

- **Figur 1 a:**: Schematische Darstellung eines Systems zur Absorptionsmessung in reflektorischer Anordnung
- **Figur 1 b:**: Schematische Darstellung eines Systems zur Bestimmung der Konzentration eines Analyten in Durchlichtdarstellung
- **Figur 2 a:**: Schematische Darstellung des Aufbaus eines Testelementes mit einem Nachweisbereich, der alternierend mehrere gleich große Reaktionsbereiche und Referenzbereiche enthält

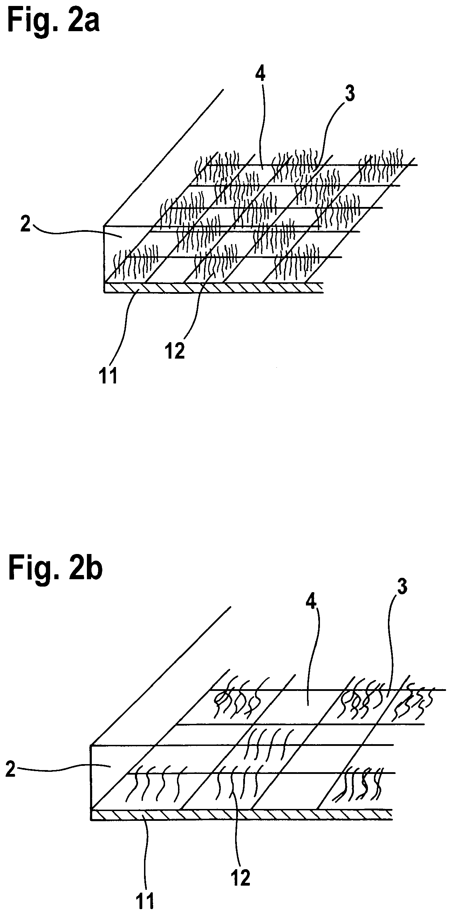

- **Figur 2 b:**: Schematische Darstellung des Aufbaus eines Testelementes mit einem Nachweisbereich, der alternierend mehrere Reaktionsbereiche und Referenzbereiche, die unterschiedliche Größe aufweisen

In den Figuren 1 a und 1b sind Systeme dargestellt, die ein Testelement (1) mit einem Nachweisbereich (2) zeigen, das mehrere Referenz (4)- und Reaktionsbereiche (3) enthält. Dies System ist nur beispielhaft mit mehreren Referenz- und Reaktionsbereichen dargestellt, es sind auch Systeme denkbar, die nur einen Referenz (4)- und einen Reaktionsbereich (3) enthalten.

In Figur 1 a ist das Testelement (1) so in dem System angeordnet, dass der Nachweisbereich (2) mindestens zum Teil durch eine Beleuchtungseinheit (6) angestrahlt wird und das vom Nachweisbereich (2) reflektierte Licht von einem Detektor (7) aufgefangen wird. Die vom Detektor (7) empfangenen Signale können in einer Auswerteeinheit (8) weiterverarbeitet und ausgewertet werden. Bevorzugter weise ist die Beleuchtung oder Detektion ortsaufgelöst, wodurch eine Differenzierung des Nachweisbereiches in Reaktionsbereiche (3) bzw. Referenzbereiche (4) stattfinden kann. In der Auswerteeinheit (8) können dann die Signale der Reaktionsbereiche (3) und der Referenzbereiche (4) ins Verhältnis gesetzt werden und mit einem Algorithmus ausgewertet werden.

In Figur 1b ist ein System dargestellt, das wiederum ein Testelement (1) sowie eine Beleuchtungseinheit (6) und einen Detektor (7) enthält, wobei der Detektor auf der gegenüberliegenden Seite vom Testelement (1) im Gegensatz zur Beleuchtungseinheit angeordnet ist. Bei dieser Ausführungsform muss gewährleistet werden, dass mindestens der Nachweisbereich des Testelementes die Strahlung durchlässt, so dass mit Hilfe des Detektors (7) die Absorption in Folge der Reaktion des Analyten mit dem Reagenz vermessen werden kann. Auch in dieser Ausführungsform wird die Beleuchtung und / oder Detektion ortsaufgelöst durchgeführt, wodurch die Reaktionsbereiche (3) und Referenzbereiche (4) voneinander unterschieden werden können. Sowohl in dem System in Figur 1A wie in Figur 1 B können zwischen dem Testelement und der Beleuchtungseinheit auf der einen Seite sowie dem Testelement und der Detektionseinheit auf der anderen Seite zusätzliche Abbildungseinheiten (5) eingesetzt werden, um möglichst effektiv das Testelement auszuleuchten, bzw. zu detektieren. Diese Abbildungseinheiten (5) können beispielsweise Linsen, Blenden oder Filter sein.

In den Figuren 2a und 2b ist der schematische Aufbau eines Nachweisbereiches (2) eines Testelementes (1) dargestellt. Vorzugsweise besitzt das Testelement (1) eine Trägerfolie (11) auf der Reagenzien (12) immobilisiert werden können. Das Reagenz (12) ist nicht über die gesamte Fläche des Nachweisbereiches (2) immobilisiert, sondern es wechseln sich Bereiche mit immobilisiertem Reagenz, die so genannten Reaktionsbereiche (3) mit Referenzbereichen (4) ab, in denen kein Reagenz immobilisiert ist.

In Figur 2A ist eine sehr regelmäßige Anordnung von Reaktionsbereichen (3) und Referenzbereichen (4) dargestellt, wohingegen in Figur 2b eine ebenfalls alternierende Variante von Reaktionsbereichen (3) und Referenzbereichen (4) dargestellt ist, bei denen jedoch die Größe der Bereiche variieren kann.

### Bezugszeichen

- 1: Testelement
- 2: Nachweisbereich
- 3: Reaktionsbereich
- 4: Referenzbereich
- 5: Abbildungseinheit
- 6: Beleuchtungseinheit
- 7: Detektor
- 8: Auswerteeinheit
- 11: Trägerfolie
- 12: immobilisierte Komponente

## Patentansprüche

1. System zur Bestimmung der Konzentration eines Analyten in einer Körperflüssigkeit durch Absorptionsmessung beinhaltend
- ein Testelement (1) mit einem Nachweisbereich (2), der mindestens einen Reaktionsbereich (3) mit Reagenzien zum Nachweis des Analyten enthält, die bei Reaktion mit dem Analyten eine Veränderung der optischen Dichte im Reaktionsbereich (3) verursachen, wobei sich im Verlauf der Reaktion ein Farbstoff bildet, der das Absorptionsverhalten des mindestens einen Reaktionsbereiches (3) verändert, und
- der Nachweisbereich (2) mindestens einen Referenzbereich (4) enthält in dem im Wesentlichen keine Veränderung des Absorptionsverhaltens durch den Analyten erfolgt, wobei mindestens der Farbstoff bildende Reaktionsschritt in dem mindestens eine Referenzbereich (4) nicht stattfindet,
- eine Detektionseinheit (7) zur räumlich aufgelösten Detektion von Lichtintensitäten, die zumindest von einem Teil des Nachweisbereiches (2) empfangen werden, der sowohl mindestens einen Reaktionsbereich (3) als auch einen Referenzbereich (4) beinhaltet, und
- eine Auswerteeinheit (8) zur Auswertung von Signalen der Detektionseinheit,
**dadurch gekennzeichnet, dass** sowohl der mindestens eine Reaktionsbereich (3) als auch der mindestens eine Referenzbereich (4) so beschaffen sind, dass sich das Absorptionsverhalten bei Benetzung im Wesentlichen nicht verändert.

2. System gemäß Anspruch 1 **dadurch gekennzeichnet, dass** der Nachweisbereich (2) mehrere Reaktionsbereiche (3) und mehrere Referenzbereiche (4) enthält.

3. System gemäß Anspruch 2 **dadurch gekennzeichnet, dass** die Reaktionsbereiche (3) und die Referenzbereiche (4) in 2 Dimensionen alternierend angeordnet sind.

4. System gemäß einem der Ansprüche 1, 2 oder 3, **dadurch gekennzeichnet, dass** der mindestens eine Referenzbereich (4) eine Beschichtung oder Imprägnierung aufweist.

5. System gemäß Anspruch 4, **dadurch gekennzeichnet, dass** die Beschichtung oder Imprägnierung eine Membran oder ein Kunststoff oder Kunstharz ist.

6. System gemäß einem der vorherigen Ansprüche, **dadurch gekennzeichnet, dass** in dem Reaktionsbereich (3) bei Anwesenheit des Analyten ein wasserunlöslicher Farbstoff entsteht.

7. System gemäß einem der vorherigen Ansprüche, **dadurch gekennzeichnet, dass** die Reagenzien mindestens ein Enzym enthalten, das mit dem Analyt reagiert.

8. System gemäß einem der vorherigen Ansprüche, **dadurch gekennzeichnet, dass** die Referenzbereiche (4) kein aktives Enzym enthalten.

9. System gemäß einem der vorherigen Ansprüche, **dadurch gekennzeichnet, dass** die Anordnung und/oder Form des Reaktionsbereiches (3) und des Referenzbereiches (4) zufällig ist.

10. System gemäß einem der vorherigen Ansprüche, **dadurch gekennzeichnet, dass** die Reaktionsbereiche (3) und Referenzbereiche (4) die gleiche Größe und das gleiche Volumen aufweisen.

11. System gemäß einem der vorherigen Ansprüche, **dadurch gekennzeichnet, dass** die Fläche des Reaktionsbereiches (3) oder des Referenzbereiches (4) kleiner als 1 mm² ist.

12. System gemäß einem der vorherigen Ansprüche, **dadurch gekennzeichnet, dass** transmittiertes und / oder reflektiertes Licht detektiert wird.

13. Testelement (1) zur Bestimmung der Konzentration eines Analyten in einer Körperflüssigkeit durch Bestimmung der optischen Dichte, beinhaltend
- einen Nachweisbereich (2), der mehrere Reaktionsbereiche (3) mit Reagenzien zum Nachweis des Analyten enthält, die bei Reaktion mit dem Analyten eine Veränderung der optischen Dichte in den Reaktionsbereichen (3) verursachen, wobei sich im Verlauf der Reaktion ein Farbstoff bildet, der das Absorptionsverhalten der Reaktionsbereiche (3) verändert, sowie mehrere Referenzbereiche (4), in denen im Wesentlichen keine Veränderung der optischen Dichte durch den Analyten erfolgt, enthält, wobei mindestens der Farbstoff bildende Reaktionsschritt in den Referenzbereichen (4) nicht stattfindet,
**dadurch gekennzeichnet, dass** sowohl die Reaktionsbereiche (3) als auch die Referenzbereiche (4) so beschaffen sind, dass sich das Absorptionsverhalten bei Benetzung im Wesentlichen nicht verändert.

14. Testelement (1) nach Anspruch 13, **dadurch gekennzeichnet, dass** mehrere Reaktionsbereiche (3) und mehrere Referenzbereiche (4) alternierend in 2 Dimensionen angeordnet sind.

15. Verfahren zur Herstellung eines Testelementes (1) zur Bestimmung eines Analyten in einer Körperflüssigkeit, wobei das Testelement (1) definiert ist wie in einem der Ansprüche 1 bis 14, mit den Schritten:
- Bereitstellen eines Trägermaterials (11)
- Aufbringen eines Nachweisbereiches (2) auf das Trägermaterial (11), wobei der Nachweisbereich (2) mindestens einen Reaktions- (3) und mindestens einen Referenzbereich (4) aufweist, wobei der mindestens eine Reaktionsbereich (3) Reagenzien zum Nachweis des Analyten enthält, die bei Reaktion mit dem Analyten eine Veränderung der optischen Dichte in dem mindestens einen Reaktionsbereich (3) verursachen, wobei sich im Verlauf der Reaktion ein Farbstoff bildet, der das Absorptionsverhalten des mindestens einen Reaktionsbereichs (3) verändert, wobei mindestens der Farbstoff bildende Reaktionsschritt in dem mindestens einen Referenzbereich (4) nicht stattfindet,
**dadurch gekennzeichnet, dass** sowohl der mindestens eine Reaktionsbereich (3) als auch der mindestens eine Referenzbereich (4) so beschaffen sind, dass sich das Absorptionsverhalten bei Benetzung im Wesentlichen nicht verändert.

16. Verfahren nach Anspruch 15 **dadurch gekennzeichnet, dass** die Reaktionsbereiche (3) und die Referenzbereiche (4) alternierend in 2 Dimensionen angeordnet sind.

17. Verfahren gemäß Anspruch 16, **dadurch gekennzeichnet, dass** die Anordnung und/oder Form des Reaktionsbereiches (3) und des Referenzbereiches (4) zufällig erfolgt.

18. Verfahren nach Anspruch 15, 16 oder 17, **dadurch gekennzeichnet, dass** zum Aufbringen von Reagenzien auf den Nachweisbereich (2) ein Druckverfahren und/oder ein Rakelverfahren benutzt werden.

19. Verfahren nach einem der Ansprüche 15 bis 18, **dadurch gekennzeichnet, dass** die Referenzbereiche (4) durch Laserbestrahlung und/oder Veränderung der chemischen Zusammensetzung von einem Teil des Reaktionsbereiches (3) erzeugt wird.

20. Verfahren nach einem der Ansprüche 15 bis 19, **dadurch gekennzeichnet, dass** mindestens ein Teil der Reagenzien in den Referenzbereichen (4), bevorzugt durch Laserbestrahlung und/oder chemischer Behandlung, deaktiviert wird.

21. Verfahren nach einem der Ansprüche 15 bis 20, **dadurch gekennzeichnet, dass** die Referenzbereiche (4) mindestens eine Substanz weniger enthalten als die Reaktionsbereiche (3).

22. Verfahren nach einem der Ansprüche 15 bis 21, **dadurch gekennzeichnet, dass** die Referenzbereiche (4) mindestens eine weitere Substanz enthalten die eine Reaktion mit dem Analyten verhindert.

23. Verfahren zum Nachweis eines Analyten mit einem Testelement (1) gemäß Anspruch 13, mit den Schritten:
- Aufbringen der Probe auf den Nachweisbereich (2)
- ortsaufgelöstes Vermessen der reflektierten Lichtintensitäten vom Nachweisbereich (2)
- Bestimmung der Analytkonzentration mit Hilfe der von mindestens einem Reaktionsbereich (3) und von mindestens einem Referenzbereich (4) vermessenen Lichtintensitäten.

24. Verfahren nach Anspruch 23, **dadurch gekennzeichnet, dass** ein Probevolumen von kleiner 500 nl aufgebracht wird.

## Claims

1. System for determining the concentration of an analyte in a body fluid by way of absorption measurement, including
- a test element (1) having a detection region (2) containing at least one reaction region (3) with reagents for detecting the analyte that cause a change in the optical density in the reaction region (3) upon reaction with the analyte, wherein, in the course of the reaction, a dye is formed that changes the absorption behaviour of the at least one reaction region (3), and
- the detection region (2) includes at least one reference region (4) in which substantially no change in the absorption behaviour due to the analyte occurs, wherein at least the dye forming reaction step does not take place in the at least one reference region (4),
- a detection unit (7) for the spatially resolved detection of light intensities received at least from a part of the detection region (2) that includes both at least one reaction region (3) and one reference region (4), and
- an evaluation unit (8) for evaluating signals of the detection unit,
**characterized in that** both the at least one reaction region (3) and the at least one reference region (4) are provided such that the absorption behaviour substantially does not change upon wetting.

2. System according to Claim 1, **characterized in that** the detection region (2) includes a plurality of reaction regions (3) and a plurality of reference regions (4).

3. System according to Claim 2, **characterized in that** the reaction regions (3) and the reference regions (4) are arranged in alternating fashion in 2 dimensions.

4. System according to one of Claims 1, 2 or 3, **characterized in that** the at least one reference region (4) has a coating or impregnation.

5. System according to Claim 4, **characterized in that** the coating or impregnation is a membrane or a plastic or a synthetic resin.

6. System according to one of the preceding claims, **characterized in that** a water-insoluble dye is formed in the reaction region (3) if the analyte is present.

7. System according to one of the preceding claims, **characterized in that** the reagents contain at least one enzyme that reacts with the analyte.

8. System according to one of the preceding claims, **characterized in that** the reference regions (4) do not contain an active enzyme.

9. System according to one of the preceding claims, **characterized in that** the arrangement and/or shape of the reaction region (3) and of the reference region (4) is random.

10. System according to one of the preceding claims, **characterized in that** the reaction regions (3) and reference regions (4) have the same size and the same volume.

11. System according to one of the preceding claims, **characterized in that** the surface area of the reaction region (3) or of the reference region (4) is smaller than 1 mm².

12. System according to one of the preceding claims, **characterized in that** transmitted and/or reflected light is detected.

13. Test element (1) for determining the concentration of an analyte in a body fluid by determining the optical density, including
- a detection region (2) containing a plurality of reaction regions (3) with reagents for detecting the analyte that cause a change in the optical density in the reaction regions (3) upon reaction with the analyte, wherein, in the course of the reaction, a dye is formed that changes the absorption behaviour of the reaction regions (3), and a plurality of reference regions (4) in which substantially no change in the optical density due to the analyte occurs, wherein at least the dye forming reaction step does not take place in the reference regions (4),
**characterized in that** both the reaction regions (3) and the reference regions (4) are provided such that the absorption behaviour substantially does not change upon wetting.

14. Test element (1) according to Claim 13, **characterized in that** a plurality of reaction regions (3) and a plurality of reference regions (4) are arranged in alternating fashion in 2 dimensions.

15. Method for producing a test element (1) for determining an analyte in a body fluid, wherein the test element (1) is defined as in one of claims 1 to 14, having the steps of:
- providing a carrier material (11),
- applying a detection region (2) onto the carrier material (11), wherein the detection region (2) includes at least one reaction region (3) and at least one reference region (4), wherein the at least one reaction region (3) contains reagents for detecting the analyte that cause a change in the optical density in the at least one reaction region (3) upon reaction with the analyte, wherein, in the course of the reaction, a dye is formed that changes the absorption behaviour of the at least one reaction region (3), wherein at least the dye forming reaction step does not take place in the at least one reference region (4),
**characterized in that** both the at least one reaction region (3) and the at least one reference region (4) are provided such that the absorption behaviour substantially does not change upon wetting.

16. Method according to Claim 15, **characterized in that** the reaction regions (3) and the reference regions (4) are arranged in alternating fashion in 2 dimensions.

17. Method according to Claim 16, **characterized in that** the arrangement and/or the shape of the reaction region (3) and of the reference region (4) is random.

18. Method according to Claim 15, 16 or 17, **characterized in that** a printing method and/or a scraper method is used to apply reagents onto the detection region (2).

19. Method according to one of Claims 15 to 18, **characterized in that** the reference regions (4) are produced by laser irradiation and/or changing the chemical composition of a part of the reaction region (3) .

20. Method according to one of Claims 15 to 19, **characterized in that** at least some of the reagents in the reference regions (4) are deactivated, preferably by laser irradiation and/or chemical treatment.

21. Method according to one of Claims 15 to 20, **characterized in that** the reference regions (4) contain at least one substance fewer than the reaction regions (3) .

22. Method according to one of Claims 15 to 21, **characterized in that** the reference regions (4) contain at least one further substance that prevents reaction with the analyte.

23. Method for detecting an analyte with a test element (1) according to Claim 13, having the steps of:
- applying the sample onto the detection region (2),
- spatially resolved measurement of the reflected light intensities from the detection region (2),
- determining the analyte concentration via the light intensities measured of at least one reaction region (3) and of at least one reference region (4).

24. Method according to Claim 23, **characterized in that** a sample volume of less than 500 nl is applied.

## Revendications

1. Système de détermination de la concentration d'un analyte dans un liquide corporel par mesure d'absorption, comprenant
- un élément de test (1) doté d'une zone de détection (2), laquelle contient au moins une zone de réaction (3) avec des réactifs servant à détecter l'analyte qui, lors de la réaction avec l'analyte, provoquent une modification de la densité optique dans la zone de réaction (3), un colorant qui modifie le comportement d'absorption de l'au moins une zone de réaction (3) se formant au cours de la réaction, et
- la zone de détection (2) contenant au moins une zone de référence (4) dans laquelle n'a lieu sensiblement aucune modification du comportement d'absorption par l'analyte, au moins l'étape de réaction formant le colorant n'ayant pas lieu dans l'au moins une zone de référence (4),
- une unité de détection (7) pour la détection avec résolution spatiale d'intensités lumineuses qui sont reçues au moins par une partie de la zone de détection (2) qui contient à la fois au moins une zone de réaction (3) et une zone de référence (4), et
- une unité d'interprétation (8) destinée à interpréter les signaux de l'unité de détection,
**caractérisé en ce qu'**à la fois l'au moins une zone de réaction (3) et l'au moins une zone de référence (4) sont réalisées de telle sorte que le comportement d'absorption ne change sensiblement pas lors de l'humidification.

2. Système selon la revendication 1, **caractérisé en ce que** la zone de détection (2) contient plusieurs zones de réaction (3) et plusieurs zones de référence (4) .

3. Système selon la revendication 2, **caractérisé en ce que** les zones de réaction (3) et les zones de référence (4) sont disposées en alternance dans 2 dimensions.

4. Système selon l'une des revendications 1, 2 ou 3, **caractérisé en ce que** l'au moins une zone de référence (4) possède un revêtement ou une imprégnation.

5. Système selon la revendication 4, **caractérisé en ce que** le revêtement ou l'imprégnation est une membrane ou une matière plastique ou de la résine synthétique.

6. Système selon l'une des revendications précédentes, **caractérisé en ce qu'**un colorant non soluble dans l'eau se forme dans la zone de réaction (3) en présence de l'analyte.

7. Système selon l'une des revendications précédentes, **caractérisé en ce que** les réactifs contiennent au moins un enzyme qui réagit avec l'analyte.

8. Système selon l'une des revendications précédentes, **caractérisé en ce que** les zones de référence (4) ne contiennent pas d'enzyme actif.

9. Système selon l'une des revendications précédentes, **caractérisé en ce que** l'arrangement et/ou la forme de la zone de réaction (3) et de la zone de référence (4) est aléatoire.

10. Système selon l'une des revendications précédentes, **caractérisé en ce que** les zones de réaction (3) et les zones de référence (4) présentent la même taille et le même volume.

11. Système selon l'une des revendications précédentes, **caractérisé en ce que** la surface de la zone de réaction (3) ou de la zone de référence (4) est inférieure à 1 mm².

12. Système selon l'une des revendications précédentes, **caractérisé en ce que** de la lumière transmise et/ou réfléchie est détectée.

13. Élément de test (1) destiné à déterminer la concentration d'un analyte dans un liquide corporel par détermination de la densité optique, comprenant
- une zone de détection (2), laquelle contient plusieurs zones de réaction (3) avec des réactifs servant à détecter l'analyte qui, lors de la réaction avec l'analyte, provoquent une modification de la densité optique dans les zones de réaction (3), un colorant qui modifie le comportement d'absorption des zones de réaction (3) se formant au cours de la réaction, ainsi que plusieurs zones de référence (4) dans lesquelles n'a lieu sensiblement aucune modification de la densité optique par l'analyte, au moins l'étape de réaction formant le colorant n'ayant pas lieu dans les zones de référence (4),
**caractérisé en ce qu'**à la fois les zones de réaction (3) et les zones de référence (4) sont réalisées de telle sorte que le comportement d'absorption ne change sensiblement pas lors de l'humidification.

14. Élément de test (1) selon la revendication 13, **caractérisé en ce que** plusieurs zones de réaction (3) et plusieurs zones de référence (4) sont disposées en alternance dans 2 dimensions.

15. Procédé de fabrication d'un élément de test (1) destiné à déterminer un analyte dans un liquide corporel, l'élément de test (1) étant défini comme dans l'une des revendications 1 à 14, comprenant les étapes suivantes :
- fourniture d'un matériau porteur (11),
- application d'une zone de détection (2) sur le matériau porteur (11), la zone de détection (2) possédant au moins une zone de réaction (3) et au moins une zone de référence (4), l'au moins une zone de réaction (3) contenant des réactifs servant à détecter l'analyte qui, lors de la réaction avec l'analyte, provoquent une modification de la densité optique dans l'au moins une zone de réaction (3), un colorant qui modifie le comportement d'absorption de l'au moins une zone de réaction (3) se formant au cours de la réaction, au moins l'étape de réaction formant le colorant n'ayant pas lieu dans l'au moins une zone de référence (4),
**caractérisé en ce qu'**à la fois l'au moins une zone de réaction (3) et l'au moins une zone de référence (4) sont réalisées de telle sorte que le comportement d'absorption ne change sensiblement pas lors de l'humidification.

16. Procédé selon la revendication 15, **caractérisé en ce que** les zones de réaction (3) et les zones de référence (4) sont disposées en alternance dans 2 dimensions.

17. Procédé selon la revendication 16, **caractérisé en ce que** l'arrangement et/ou la forme de la zone de réaction (3) et de la zone de référence (4) s'effectue de manière aléatoire.

18. Procédé selon la revendication 15, 16 ou 17, **caractérisé en ce qu'**un procédé d'impression et/ou un procédé à la spatule est utilisé pour l'application des réactifs sur la zone de détection (2).

19. Procédé selon l'une des revendications 15 à 18, **caractérisé en ce que** les zones de référence (4) sont générées par irradiation au laser et/ou modification de la composition chimique d'une partie de la zone de réaction (3).

20. Procédé selon l'une des revendications 15 à 19, **caractérisé en ce qu'**au moins une partie des réactifs est désactivée dans les zones de référence (4), de préférence par irradiation au laser et/ou traitement chimique.

21. Procédé selon l'une des revendications 15 à 20, **caractérisé en ce que** les zones de référence (4) contiennent au moins une substance de moins que les zones de réaction (3).

22. Procédé selon l'une des revendications 15 à 21, **caractérisé en ce que** les zones de référence (4) contiennent au moins une substance supplémentaire qui empêche une réaction avec l'analyte.

23. Procédé de détection d'un analyte avec un élément de test (1) selon la revendication 13, comprenant les étapes suivantes :
- application de l'échantillon sur la zone de détection (2),
- mesure avec résolution spatiale des intensités lumineuses réfléchies de la zone de détection (2),
- détermination de la concentration d'analyte à l'aide des intensités lumineuses mesurées par au moins une zone de réaction (3) et par au moins une zone de référence (4).

24. Procédé selon la revendication 23, **caractérisé en ce qu'**un volume d'échantillon inférieur à 500 nl est appliqué.
